# EUROPEAN PATENT APPLICATION

(11) **EP 1 589 464 A2**
(43) Date of publication of application: **26.10.2005**
(21) Application number: 05008606.5
(22) Date of filing: 27.01.2004
(51) Int. Cl.: G06F 19/00, A61B 5/0432

(54) **Transtelephonic multifunctional electrocardiograph**

(30) Priority: 30.01.2003 IT MI20030160
(62) Divisional of application: 04001708.9
(71) Applicant: Sgalambro, Gaetano, 37045 Legnago (VR) (IT)
(72) Inventor: Sgalambro, Gaetano, 37045 Legnago (VR) (IT)
(74) Representative: La Ciura, Salvatore

(57) **Abstract**

This application proposes a multifunctional diagnostic device and in particular an intermittent dynamic electro-cardiographic device with smart control and telematic transmission.

More in particular it relates to a diagnostic device with different sensors which can collect different biological signals such as heart rate, arterial pressure etc., an analogic/digital converter for sensor signals, a portable signal receiver which can analyse and process such data and a device to activate tele-transmission via GSM or other to the central server of all or part of the data.

The analogic/digital converter is placed close to the sensors so that the signals can be converted immediately on reception and transmitted digitally which is easier to manage and significantly reduces external interference.

## Description

This invention is a smart multifunctional portable patient terminal (with a built-in diagnostic algorithm).

It is part of a telematic cardiological multi-diagnostic system with a central server and an smart data receiving computer in the cardiologist's office.

The patient recorder is flexible and can record several kinds of biological signal (ECG, external arterial pressure) thanks to its special technical features and interchangeable units.

The following description includes an example of the patient terminal recording electrical signals produced by cardiac activity defined as an electrode unit.

This patient terminal is part of a dynamic electro cardiographic diagnostic system, a development of the first "dynamic device" by Dr. N.J. Holter in 1959.

This system consisted of 2 units, a portable recorder and a desktop cardiological reader/analyser.

A patient recorder consisting of a multi-wire cable with three disposable electrodes to be applied to the patient's chest, recording the bioelectrical cardiac signals on magnetic tape.

A desk top analyser consisting of a tape-deck for high speed play back and a graphic system for printing out the signals with a control system.

The recording time was six hours continuous recording on a single channel.

The diagnostic analysis was a time consuming procedure.

The Holter system went into production when its clinical usefulness became apparent and its performance and technology were continuously updated and improved.

By the mid-'90 the system featured:
- a compact portable device with:
   24 hour memory (C60 magnetic tape)
   3 channels
- desk top device with:
   fast play-back (240xreal time)
   automatic signal analysis

In the meantime, electronic RAM recorders were being developed but with limited memory.

This meant it was necessary to compress data.

There are several ways of doing this:
- by reducing the sampling frequency to 100mhz
- or by recording a single channel
- or by smart statistical compression and sampling
- and/or automatic data analysis.

This technique of automatic processing only memorizes final numerical data, thus extending memory capacity.

Sampling classifies the electrical waves into families and records a single sample and totals the number of impulses/family.

In both cases there are, of course, advantages and disadvantages.

They require smaller diagnostic algorithms because they operate in real time and not at 240x i.e. play-back time, but they do not record all the original signals.

This electronic RAM device is suitable for intermittent dynamic recordings activated by the patient.

Later developments included trans-telephonic transmission via modem or acoustic coupling to a central cardiological unit.

Technological progress has lead to high density RAM and widespread systems integration overcoming historical system limits.

At the same time medical interest in telematic solutions and especially telecardiological and intermittent dynamic trans-telephonic electro-cardiographic devices grew.

Such dynamic device have limits due to their project and recording techniques.

The advantages of continuous 24hour Holter is beat by beat analysis but it produces an enormous quantity of data requiring a large investment in high-tech data analysis equipment.

The advantage of intermittent trans-telephonic recording is almost real-time analysis but of only a few sample data.

There may be problems with the quality of the electrical signals such as background interference noise due to patient activity or poor skin/electrode contact or the length of the cabling..

These noises may create false pathological events.

Good quality electrocardiographical signals improve the reliability of the diagnostic algorithm which is the main objective in clinical practice.

The main feature of this invention is that the biological signals are recorded, processed by standard procedures and converted immediately into digital data within the electrode unit which is physically independent and connected to the main unit by a single cable.

This digital connection makes it possible to interchange the recording modules and create a poly-functional unit i.e. 4 or 12 channel ECG, arterial pressure and other biological function recorders

The digital data are therefore less subject to external interference, so they are better processed by the software in the recording unit and algorithmic errors are considerably reduced, compared to traditional systems.

In statistical terms the existing algorithms are more sensitive and specific.

In technical terms the cardiac bioelectrical signals have a much improved noise/signal interference ratio.

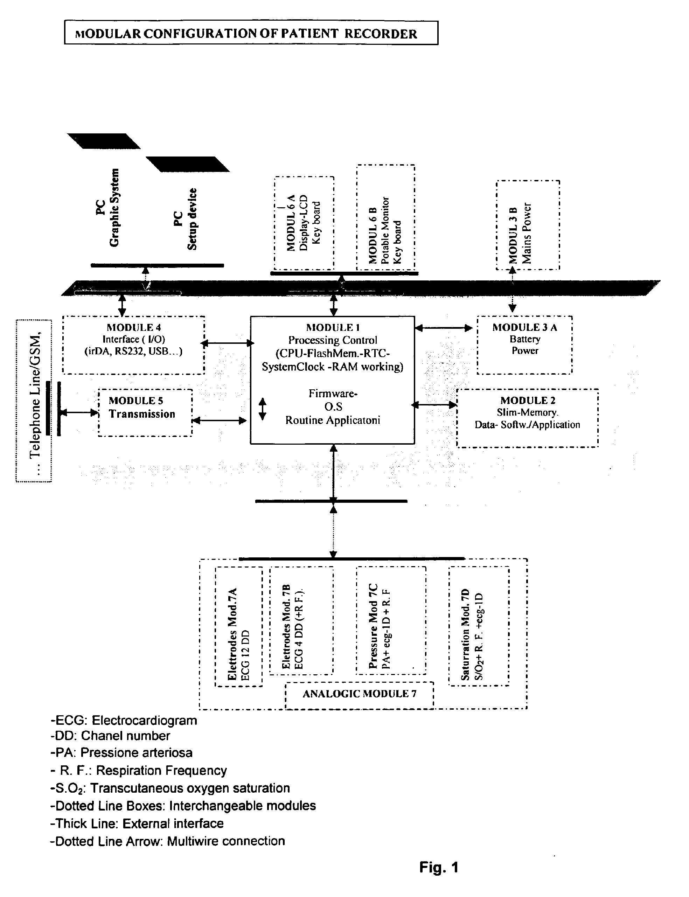

For a detailed description see figures 1) to 3)
Figure 1) shows the flexible modular configuration of the patient recorder invention.

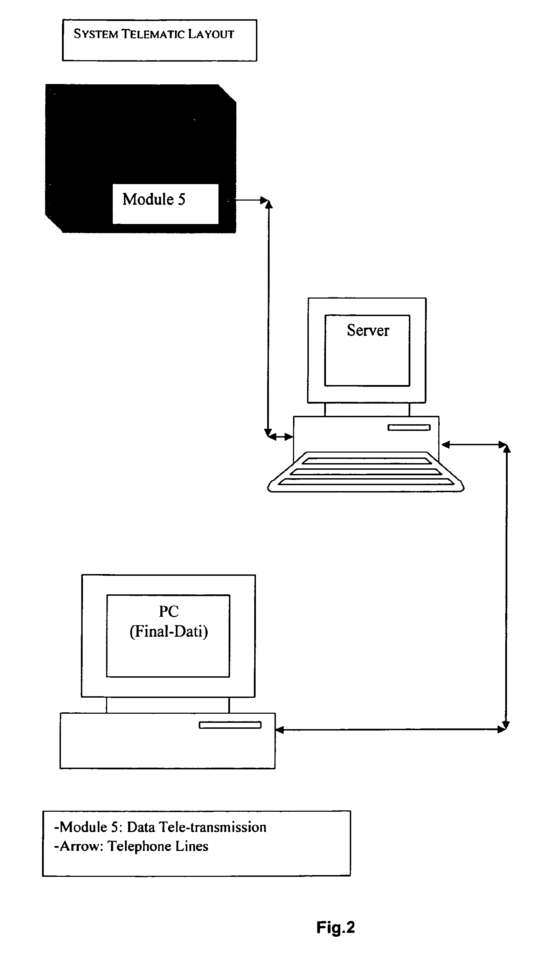

Figure 2) shows the complete diagnostic telematic system layout including the patient recorder.

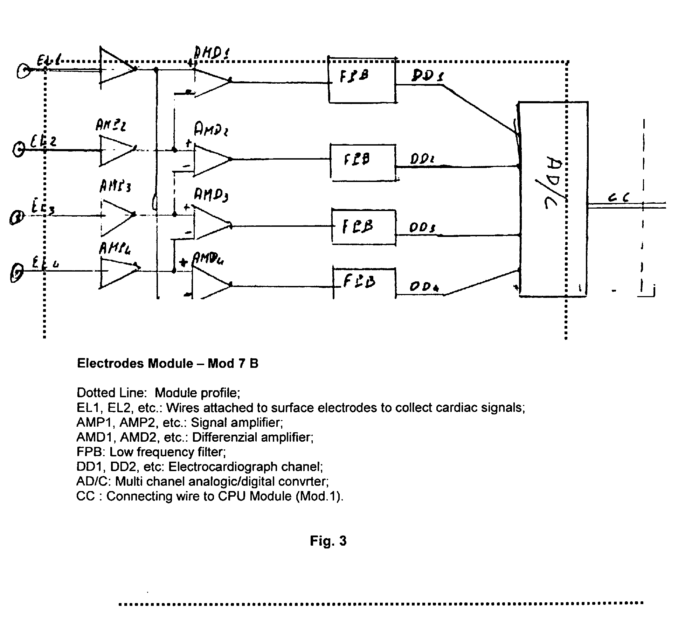

Figure 3) shows the electrical unit layout (Mod. 7B Fig.1).

The compact, portable patient recorder in this invention is an integrated electronic microprocessor system with static working RAM, data programme slim card, input/output unit, tele-transmission unit, LCD graphic display, battery and mains powered mini-keyboard.

It consists of seven physically independent inter-changeable modules:
1) CPU module
2) Slim memory module
3) Power module
4) Interface module
5) Transmission module
6) Display/Key board module
7) Analogic module

The modules are inter-connected by connectors and/or wires and can be selected according to the function(s) required.

This is the main feature of the invention in question.

Five of these modules are built in the patient recorder and two are external (see Fig. 1)

The CPU module is connected to all the other modules by a BUS interface.

It consists of:
- A CPU with flash memory and "on chip".
- RAM, static working.
- RAM loads application software slim-memory (Mod.2) and continuously updates the recorded electro-cardiographic signals and diagnostic processing.
- Real time clock
- Systems clock
- BUS: addresses data base, BUS interface modules, BUS memory selection, BUS control signals
- BUS decoder, memory selection
- Stand-by battery for back-up RAM
- Power control

On-chip memory with firmware, an operation/application system, with specific sub-routine software controls and manages the functions of all units.

The module is built according to standard technologies.

The slim memory module contains the application programme for the current diagnostic function e.g. diagnostic processing programme, medical recording and patient recorder interfaces etc.

The significant data memory is divided into three data banks:
1. patient activated ECG signals
2. algorithmic activated diagnostic signals
3. diagnostic processing data.

The power module: rechargeable or disposable batteries. It powers the entire system via the CPU module.

The power network and digital networks are independent but can use the same connectors.

The interface module can be in series RS232 or IrDA, USB wireless. The doctor can thus check the patient recorder set up.

The transmission module (Mod.5) is a telephone modem on a general purpose PCMCIA. It transmits data in the slim memory to the central server. The transmission protocol is loaded in the slim memory and managed by Module 1. Transmission is activated either by the patient or by the diagnostic algorithm according to previously defined pathological criteria.

The display/Keyboard module (Mod.6) is physically connected to the recording module by a cable. It consists of an LCD display unit and 5key-mini keyboard. It manages the communication between doctor/patient (set up) and patient/terminal communication. It may be replaced by a portable monitor for bed-ridden patients.

The Analogic module (Mod.7) collects the different biological signals via specific sensors, processes them and sends them to the CPU (Mod.1) via a connecting cable (see fig. 1).

The patient recorder is an integral part of a poly-diagnostic telematic system which connects the patient directly with his doctor. The data transmitted from the patient recorder are received automatically by the central server which stores them and prepares a standard report which is then sent in real time to the doctor's PC.

A particular feature of this invention is the Analogic module (Mod. 7B) in Fig.1. It consists of a mini casing containing the electronics and cardiac bioelectric processing technology, an AD/C multi-channel converter and a 4 channel input connected by wires (EL1, EL2, EL3, EL4) to the surface electrodes on the patient's chest and by another wire to the patient terminal and by a multi-wire (CC) which connects to the patient terminal.

The CC multi-wire transmits the digital signals from the Analogic module to the CPU (Mod. 1).

The Analogic module is powered and managed by the CPU. A specific feature here is a converter which sends digital cardiac bioelectric signals to the CPU (Mod.1) with an optimum signal/interference noise ratio.

The advantages of this technique are:
- increased efficiency/efficacy of cleaning software e.g. "digital signal averaging".
- Improved quality of final cardiac bioelectric signals both for automated diagnosis and direct cardiological diagnosis.

The increased reliability for automated diagnosis thanks to the improved signal/noise interference ratio of this invention can be coupled with a smart real time tele-transmission procedure to create a "smart trans-telephonic telemetry" for continuous observation without human intervention and continuous line connection.

In fact, the continuous observation is carried out by the diagnostic algorithm which activates the memory and on-line transmission via the server when there is a pre-defined pathological situation.

All the data are continuously recorded in the slim memory module (Mod.2) until required for any direct analysis.

### PROCEDURE

When the patient recorder is switched on, the CPU loads the application from the slim memory module (Fig.1 Mod.2), into the RAM ready for medical set up and connection to patient.

When monitoring starts, the signals are continuously collected and digitalized in the Analogic module, recorded in RAM and then processed in the CPU, "cleaned" by the D.S.A and then processed according to the diagnostic algorithms and prepared for transfer to the slim memory for storing.

There are two simultaneous storage procedures:
- Continuous storage of diagnostic data and electro-cardiographic signals.
- Intermittent storage of patient activated and/or diagnostic algorithm activated ECG recordings.

The "on-command" recordings are transmitted to the transmission module in real time and sent to the central server which edits an on-line alphanumeric graphical report.

The continuously recorded data can be analyzed when required by direct reading via driver from slim memory.

This invention eliminates the costs associated with traditional telemetry, technical/device transmission problems via radio frequency, continuous human observation at monitor and other operating costs.

The transmission module flexibility guarantees multi-system communication.

The inter-changeability of the different modules ensures personalized patient observation according to diagnostic requirements and logistics.

## Claims

1. Dynamic electrocardiograph device comprising a module with sensors of biological parameters to be applied to the patient, a device unit apt to receive through a cable and process the signal generated by the said module and transmitting means apt to transmit by telephone the results of this processing to a central server, **characterized in that** an analogical/digital converter is positioned upstream said processing unit and **in that** said module with sensors of biological parameters is interchangeable.

2. Dynamic electrocardiograph according to claim 1, **characterized in that** said analogical/digital converter is positioned in said module with sensors of biological parameters.

3. Dynamic electrocardiograph according to claim 2, **characterized in that** the said transmitting means consist of devices apt to transmit messages with the multi-network technology.

4. Dynamic electrocardiograph according to claim 2, **characterized in that** means are provided for the manual activation of the recording and the data transmission by the patient.

5. Dynamic electrocardiograph according to claim 2, **characterized in that** means are provided for activating the transmission either by the patient or by the diagnostic algorithm according to previously defined pathological criteria.

6. Dynamic electrocardiograph according to any of the above claims, comprising:
• one or more biological parameter sensors to be applied to the patient.
• an AD/C converter associated with and close to said sensors;
• a processing device carried by the patient, which receives the signals from said analogical/digital converter
• means, controlled by said processing device apt to transmit by telephone the processing results to a central server; and
• means apt to compare the data coming from said sensors with a series of reference data previously stored in said processing system and to activate the recording of said data and/or their transmission to a central server according to the results of said comparison.
